# EUROPÄISCHE PATENTANMELDUNG

(11) **EP 4 563 109 A1**
(43) Veröffentlichungstag der Anmeldung: **04.06.2025**
(21) Anmeldenummer: 24213144.9
(22) Anmeldetag: 15.11.2024
(51) Int. Cl.: A61B 18/14, A61B 18/12, A61B 18/00

(54) **VORRICHTUNG, VERFAHREN UND SYSTEM ZUR IRREVERSIBLEN ELEKTROPORATION VON GEWEBE**

(30) Priorität: 30.11.2023 DE 102023133549
(71) Anmelder: Stockert GmbH, 79111 Freiburg im Breisgau (DE)
(72) Erfinder: KEES, Fabian, 79110 Freiburg (DE); DE FREITAS, Thomas, 79115 Freiburg (DE); STORK, David, 79114 Freiburg (DE)
(74) Vertreter: Frenkel, Matthias Alexander

(57) **Zusammenfassung**

Die vorliegende Erfindung beschreibt eine Vorrichtung, Systeme und ein Verfahren, die irreversible Elektroporation mittels energiekontrollierter Steuerung realisieren. Ein Ausführungsbeispiel der Vorrichtung weist einen elektrischen Signalgenerator, ein Elektrodenpaar und eine mit dem Signalgenerator verbundene Auswerte- und Steuereinheit auf.

## Beschreibung

Hier werden eine Vorrichtung, ein Verfahren und ein System zu irreversiblen Elektroporation von Gewebe vorgestellt. Merkmale und Eigenschaften der Vorrichtung, des Systems und des Verfahrens sind in den Ansprüchen definiert; aber auch die Beschreibung und die Figuren offenbaren Charakteristika der Vorrichtung, des Systems und des Verfahrens sowie deren unterschiedliche Aspekte und Zusammenhänge.

Gewebebehandlungen mittels gepulster elektrischer Felder hat in den letzten Jahren als klinische Anwendung sukzessive an Bedeutung gewonnen. Die Auswirkungen der dabei genutzten kurzen Hochspannungsimpulse und der entstehenden hohen elektrischen Feldstärken auf/in Gewebe sind hingegen seit über vier Jahrzehnten Bestandteil diverser Forschungsvorhaben. Eine solche Anwendung kann als nicht-thermisches Verfahren klassifiziert werden und basiert auf der Abgabe von kurzen hohen Pulsen auf/in Gewebe, um ein lokal hohes elektrisches Feld zu erzeugen, die typischerweise im Bereich von mehreren Hunderten Volt pro Zentimeter liegen können. Dadurch werden Poren in den Zellmembranen des Gewebes erzeugt. Übersteigt dieses elektrische Feld bei der Porenbildung an den Lipiddoppelschichten der Zellmembran einen gewissen Schwellwert, so kann die sogenannte Elektroporation irreversibel sein und die Poren bleiben dauerhaft geöffnet, was schließlich zur Apoptose (programmierter Zelltod) der Zelle führt.

Irreversible Elektroporation (IRE) ist ein überwiegend nicht-thermisches Verfahren, das eine Erhöhung der Gewebetemperatur um höchstens einige Grad für einige Millisekunden bewirkt. Damit unterscheidet sich IRE von der herkömmlich eingesetzten RF-Ablation (Hochfrequenz/Radiofrequency-Ablation), bei der die Gewebetemperatur um 20 bis 70 °C ansteigt und die Zellen durch Erhitzung zerstört werden. Bei der IRE werden typischerweise bipolare Pulse verwendet, d. h. Kombinationen aus positiven und negativen Pulsen, um Muskelkontraktion, die sich durch Anlegen von Gleichspannungen typischerweise ergeben, möglichst weitgehend zu vermeiden. Die Impulse können beispielsweise zwischen zwei bipolaren Elektroden eines Katheters angelegt werden oder aber zwischen einer Katheter-Elektrode und einer Körperoberflächenelektrode, die typischerweise auf die Haut des Patientenrückens geklebt wird.

Damit die IRE-Pulse die gewünschten Poren in den Zellmembranen des Zielgewebes erzeugen, muss die durch die Pulse definierte elektrische Feldstärke E am/im Zielgewebe zwischen einem Paar aus mindestens zwei Elektroden einen gewebeabhängigen Schwellenwert Etn überschreiten. So liegt der Schwellenwert für Herzzellen beispielsweise bei etwa 500 V/cm, während er für Knochen bei 3000 V/cm liegt. Diese Unterschiede in den Schwellwerten der Feldstärken ermöglichen eine selektive Anwendung von IRE in verschiedenen Geweben oder Mischgewebe (Fettgewebe, myokardiales Gewebe und Nervengewebe). Um die erforderliche Feldstärke zu erreichen, hängt die an ein Elektrodenpaar anzulegende Spannung sowohl von der Art des Zielgewebes als auch vom Abstand zwischen den Elektroden und von der Elektrodengröße selbst ab. Gleichermaßen beeinflussen diese Parameter auch den thermischen Energieeintrag während der Ablation und damit die Temperaturspitzen, die an dem zu behandelnden Gewebe auftreten können. Die angelegten Spannungen können bis zu 2000 V erreichen, was wesentlich höher ist als die typischen Spannungen von 10-200 V bei der thermischen RF-Ablation.

Der bipolare Pulsed Field Ablation-Puls (der bipolare PFA-Puls) für IRE umfasst einen positiven und einen negativen Puls, die zwischen zwei Elektroden mit einer Pulsbreite von 1 bis 5 µs und einem Abstand zwischen den positiven und negativen Pulsen von 1 bis 5 µs angelegt werden. Die bipolaren Pulse werden zu Pulsfolgen zusammengesetzt, wobei jede Folge über hundert bipolare Pulse mit einem Puls-zu-Puls-Abstand von 1 bis 10 ms umfassen kann. Die Pulsfolgen bilden jeweils einen Burst, wobei sich das gesamte Pulspaket der IRE-Ablation aus 1 bis 20 Bursts/Bursteinheiten zusammensetzt, die jeweils einen Burst-zu-Burst-Abstand von 1 bis 1000 ms aufweisen. Die Gesamtdauer einer Ablation kann bis zu 10 s betragen.

Die dargelegten Parameter des Puls-Protokolls sollten im Vorfeld der Ablation derart eingestellt werden, dass der gewünschte Elektroporationseffekt und eine damit verbundene klinische Wirksamkeit erzielt und gleichzeitig mögliche Risiken wie beispielsweise Muskelkontraktion oder eine thermische Schädigung des Gewebes vermieden werden. Neben den zeitlichen und quantitativen Variablen des Puls-Protokolls sind dabei auch die elektrischen Parameter von entscheidender Bedeutung.

Bislang erfolgt die Einstellung der elektrischen Parameter in Vergleichssystemen durch Vorgabe eines Ziel-Stroms, der zwischen mindestens zwei Elektroden durch das zu behandelnde Gewebe fließt und damit ein elektrisches Feld lokal induziert. Die Größe dieses induzierten elektrischen Feldes ist dabei abhängig von der elektrischen Impedanz des Gewebes. Gleichermaßen schwankt diese Gewebe-Impedanz allerdings je nach Position bzw. Größe der Elektroden sowie in Abhängigkeit des Patienten und damit schlussendlich auch die Energie, die durch die Prozedur in das Gewebe übertragen wird und dieses lokal erwärmen kann. Somit ist durch diese Regelung des Ziel-Stroms nicht gewährleistet, dass es zu lokal unerwünschten thermischen Effekten kommen kann, wie beispielsweise zur Bildung von Blasen an den Elektroden oder zur Verkohlung des Gewebes.

Ein weiterhin entscheidender Faktor zur Steuerung der PFA-Pulse ist die Abhängigkeit der elektrischen Gewebe-Impedanz von der anliegenden elektrischen Feldstärke E, da diese unmittelbar die Leitfähigkeit des Gewebes und damit auch den lokalen Energieeintrag beeinflusst. Geschieht demnach die Messung der elektrischen Gewebe-Impedanz bei einer anderen elektrischen Feldstärke als die eigentliche IRE-Ablation, so unterscheidet sich folglich auch der tatsächliche Energieeintrag in das Gewebe während der Ablation.

Vergleichssysteme verwenden hier zur Kompensation teilweise eine aktive Regelung während der Ablation, um den Energieeintrag zu regulieren, allerdings ist hierbei nicht gewährleistet, dass beispielsweise während des Einregelns zu Beginn der Ablation nicht bereits ein thermischer Schaden entstanden ist.

Aus der WO 2022/164750 A1 sind spannungsgesteuerte Pulssequenzen für IRE-Systeme bekannt. Ein offenbartes System weist einen Ablationskatheter mit Katheterelektrode auf. Die Katheterelektrode erzeugt im Zielgewebe ein elektrisches Feld. Eine zusätzliche Steuerung ist dazu konfiguriert, eine erste Impulsspannung einer ersten Impulssequenz zu empfangen und auf Basis der ersten Impulsspannung eine Ladespannung zu bestimmen. Ein zusätzlicher Generator ist dazu konfiguriert, eine zweite Impulsfolge mit gesteuerter Impulsspannung abzugeben.

Aus der US 2021/0228260 A1 sind ein System und ein Verfahren für eine anpassbare Wellenform und Steuerung zur Ablation mit gepulsten elektrischen Feldern bekannt. Das Verfahren offenbart unter anderem die folgenden Schritte: Konfigurieren eines ersten Ausgangstherapieparametersatzes unter Verwendung eines ausgewählten Therapieprofils; Erzeugen einer oder mehrerer erster Therapieausgaben unter Verwendung des ersten Ausgangstherapieparametersatzes; Erfassen eines oder mehrerer erster Rückkopplungsparameter; Vergleichen der ersten Rückkopplungsparameter mit einem erwarteten Rückkopplungsparameter, um ein oder mehrere erste Vergleichsergebnisse zu erzeugen, wobei der erwartete Rückkopplungsparameter mit dem ausgewählten Therapieprofil verbunden ist; und Konfigurieren eines zweiten Ausgangstherapieparametersatzes unter Verwendung der ersten Vergleichsergebnisse.

Aus der EP 3 964 153 A1 ist ein Verfahren zur impedanzbasierten ireversiblen Elektroporation bekannt. Das Verfahren umfasst das Messen einer Gewebeimpedanz und das Berechnen eines Impedanz-Schwellwertes. Der Impedanz-Schwellwert, der jedem ausgewählten Protokoll innewohnt, wird anhand von Protokollparametern berechnet, oder aus einer vorbestimmten Referenz gelesen, bspw. aus einer empirischen Referenz oder vorberechneten Referenzen, die in einer *"look-up"-* Tabelle gespeichert sind. Um die optimale Energie für den Ablationsprozess zu erreichen, wird auf Basis der gemessenen Gewebeimpedanz das Protokoll angepasst, genauer gesagt die Pulsdauer und/oder die Anzahl der Pulse und/oder die Anzahl der Bursts. Die Beträge der Spannungsspitzen werden typischerweise nicht reduziert, wenn das Protokoll angepasst wird.

Aus der US 2011 238 056 A1 sind ein System und ein Verfahren zur impedanzvermittelten Steuerung der Leistungsversorgung für die Elektrochirurgie bekannt. Das System und das Verfahren offenbaren eine Impulsserie mit einem Anfangsimpuls, dessen Profil einen voreingestellten Radiofrequenzstartwert entspricht. Beginnend mit dem Radiofrequenzstartwert steigt ein Radiofrequenzpegel mit einer Rampenrate bis zu einem voreingestellten Radiofrequenzwert.

Des Weiteren sind die Dokumente EP 3 232 967 A1, US 2022/0313346 A1, US 2007/0078453 A1, WO 2022/173875 A1, WO 2022/258034 A1, WO 2020/097276 A1 bekannt.

In Anbetracht des genannten Stands der Technik besteht weiter das Problem der Kontrolle der Energieabgabe in das zu behandelnde Gewebe im Vorfeld einer IRE-Prozedur. Insbesondere sollte es nicht nötig sein, eine Regelschleife während der Energieabgabe implementieren zu müssen, um etwaig unerwünschte thermische Effekte beispielsweise während des Einregelns zu vermeiden.

Zur Lösung dieses Problems werden eine Vorrichtung nach Anspruch 1, ein Verfahren nach Anspruch 7 und Systeme nach Anspruch 4, 5 und 6 vorgeschlagen.

Gemäß einem ersten Aspekt wird eine Vorrichtung zur gewebetypselektiven irreversiblen Elektroporation eines Gewebes vorgeschlagen. Die Vorrichtung weist einen elektrischen Signalgenerator auf. Der elektrische Signalgenerator ist dazu eingerichtet, ein elektrisches Signal gemäß einem zu empfangenden Signal-Protokoll zu generieren und zu senden. Die Vorrichtung weist ein Elektrodenpaar auf, beispielsweise genau ein einziges Elektrodenpaar. Das Elektrodenpaar ist mit dem elektrischen Signalgenerator (elektrisch) verbunden. Das Elektrodenpaar ist dazu eingerichtet, das elektrische Signal zu empfangen. Das Elektrodenpaar ist dazu eingerichtet, eine elektrische Verbindung über das zwischen dem Elektrodenpaar liegende Gewebe zu schließen. Die Vorrichtung weist eine Auswerte- und Steuereinheit auf. Die Auswerte und Steuereinheit ist mit dem Signalgenerator (elektrisch) verbunden. Die Auswerte- und Steuereinheit ist dazu eingerichtet, insbesondere in einer ersten Betriebsphase, dem Signalgenerator ein Mess-Signal-Protokoll zu senden. Die Auswerte- und Steuereinheit ist dazu eingerichtet, insbesondere in einer ersten Betriebsphase, zumindest ein durch das Gewebe gesendete Mess-Signal zu empfangen. Die Auswerte- und Steuereinheit ist dazu eingerichtet, insbesondere in einer ersten Betriebsphase, auf Basis des zumindest einen empfangenen Mess-Signals eine Gewebeimpedanz, beispielsweise genau eine einzige Gewebeimpedanz, zu bestimmen. Die Auswerte- und Steuereinheit ist dazu eingerichtet, insbesondere in einer zweiten Betriebsphase, auf Basis der bestimmten Gewebeimpedanz zumindest ein Format (z. B. von einer Vielzahl von Formaten) eines (durch einen Nutzer vordefinierten) Burst-Signalfolge-Protokolls anzupassen, um (mittels des angepassten Burst-Signalfolge-Protokolls) einen Energiebetrag pro Burst (in Joule) (dem Signalgenerator) vorzugeben/zu definieren.

Dies hat den Vorteil, dass Schwankungen in dem Energiebetrag pro Burst, die durch Gewebeimpedanz und/oder Elektrodengeometrie entstehen, im Vorfeld zur eigentlichen Ablation (Anwendung einer Burst-Signalfolge am Gewebe) kompensiert werden.

Das gesendete Mess-Signal kann einen Mess-Signalpegel, insbesondere ersten Spannungspegel, aufweisen, der einem Burst-Signalfolgepegel, insbesondere einem zweiten Spannungspegel, gleicht.

Dies hat den Vorteil, dass die Gewebeimpedanz explizit mit der gleichen anliegenden Spannung gemessen wird, mit der auch darauffolgend die Ablation durchgeführt werden soll. Da die Impedanz in Abhängigkeit der anliegenden Spannung steht, wird auf diese Weise sichergestellt, dass die tatsächlich vorhandene Impedanz bei der späteren Ablation festgestellt wird und darauf basierend können die Formate angepasst werden.

Das Elektrodenpaar kann genau ein einziges Elektrodenpaar aufweisen oder als genau ein einziges Elektrodenpaar ausgebildet sein. Die Gewebeimpedanz kann genau eine einzige Gewebeimpedanz aufweisen oder als genau eine einzige Gewebeimpedanz ausgebildet sein.

Der Signalgenerator kann als Spannungsquelle, insbesondere als Hochspannungssignal-Generator ausgebildet sein. Der Signalgenerator kann dazu eingerichtet sein, Hochspannungs-Gleichstrom (DC) pulsed field ablation (PFA) Pulse abzugeben.

Die Auswerte- und Steuereinheit kann dazu eingerichtet sein, insbesondere in einer ersten Betriebsphase, zumindest einmalig/zumindest zweimalig/vielfach einen elektrischen Strom und eine elektrische Spannung zu messen, und daraus zumindest eine/zumindest zwei/eine Vielzahl (von) (durchschnittliche(n)) Gewebeimpedanz(en) zu bestimmen.

Die Auswerte- und Steuereinheit kann dazu eingerichtet sein, insbesondere in einer zweiten Betriebsphase, auf Basis der bestimmten Gewebeimpedanz zumindest eine Kombination von (zumindest zwei) Formaten (einer Vielzahl von Formaten) eines Burst-Signalfolge-Protokolls anzupassen, um einen Energiebetrag pro Burst vorzugeben/zu definieren. Der vorgegebene/definierte Energiebetrag pro Burst kann einen Schwellwert einer in/an das Gewebe induzierten elektrischen Feldstärke überschreiten, die notwendig ist, um eine irreversible Elektroporation zu erreichen.

Mit anderen Worten kann die Auswerte- und Steuereinheit zeitliche- und/oder elektrische Parameter des elektrischen Signals (mittels eines Protokolls) kontrollieren/steuern.

Die Auswerte- und Steuereinheit kann in dem Signalgenerator angeordnet sein.

Die Auswerte- und Steuereinheit ist dazu eingerichtet, insbesondere in einer dritten Betriebsphase, dem Signalgenerator das angepasste Burst-Signalfolge-Protokoll zu senden.

Der vorgegebene/definierte Energiebetrag pro Burst kann, insbesondere in der dritten Betriebsphase, eine elektrische Feldstärke in/an das, insbesondere myokardiale, Gewebe induzieren. Der vorgegebene/definierte Energiebetrag pro Burst kann größer sein als eine für eine, insbesondere irreversible, Gewebeporung notwendige elektrische Feldstärke.

Die Formate des Burst-Signalfolge-Protokolls können Eigenschaften und/oder den Energiebetrag pro Burst einer durch den Signalgenerator zu generierende Burst-Signalfolge vorgeben.

Das/Die Format/e kann/können aufweisen: eine erste Anzahl an Bursts innerhalb der Burst-Signalfolge, zumindest einen ersten zeitlichen Abstand zwischen zumindest zwei aufeinanderfolgenden Bursts der Burst-Signalfolge, eine zweite Anzahl von bipolaren Pulsen innerhalb eines Bursts, zumindest einen zweiten zeitlichen Abstand zwischen zumindest zwei aufeinanderfolgenden bipolaren Pulsen innerhalb eines Bursts, einen dritten zeitlichen Abstand zwischen einem positiven und negativen Puls zumindest eines bipolaren Pulses, eine Pulsbreite eines positiven und/oder eines negativen Pulses zumindest eines bipolaren Pulses, einen Wert einer Pulsauslenkung eines positiven und/oder eines negativen Pulses zumindest eines bipolaren Pulses.

Die Auswerte- und Steuereinheit kann dazu eingerichtet sein, insbesondere in einer zweiten Betriebsphase, auf Basis der einen bestimmten Gewebeimpedanz zumindest ein (von einer Vielzahl von) Format(en) eines (durch einen Nutzer vordefinierte) Burst-Signalfolge-Protokolls anzupassen, um einen Energiebetrag pro Burst in Abhängigkeit des ersten zeitlichen Abstands vorzugeben/zu definieren.

Eine erste Anzahl an Bursts innerhalb der Burst-Signalfolge kann in einem Wertebereich von 1 bis 100 Bursteinheiten liegen.

Zumindest ein erster zeitlicher Abstand zwischen zwei aufeinanderfolgenden Bursts der Burst-Signalfolge kann in einem Wertebereich von 1 ms bis 1000 ms liegen.

Eine zweite Anzahl von bipolaren Pulsen kann innerhalb eines Bursts in einem Wertebereich von 1 bis 300 bipolaren Pulseinheiten liegen.

Zumindest ein zweiter zeitlicher Abstand zwischen zumindest zwei aufeinanderfolgenden bipolaren Pulsen innerhalb eines Bursts kann in einem Wertebereich von 1 bis 10 ms liegen. Ein dritter zeitlicher Abstand zwischen einem positiven und negativen Puls kann in einem Wertebereich zwischen 1 bis 5 µs liegen.

Eine Pulsbreite eines positiven und/oder eines negativen Pulses kann in einem Wertebereich zwischen 1 bis 10 µs liegen. Eine Pulsbreite eines positiven Pulses kann sich von einer Pulsbreite eines negativen Pulses unterscheiden.

Ein Wert einer Pulsauslenkung eines positiven Pulses kann in einem Wertebereich von 200 bis 2000 V liegen. Ein Wert einer Pulsauslenkung eines negativen Pulses kann in einem Wertebereich von -200 bis -2000 V liegen.

Gemäß einem zweiten Aspekt wird ein System zur irreversiblen Elektroporation eines Gewebes vorgeschlagen. Das System weist eine Vorrichtung gemäß dem ersten Aspekt und einen monopolaren Katheter auf. Der Katheter weist ein distales Ende auf. Das Elektrodenpaar ist als eine erste Elektrode und eine Köperoberflächenelektrode ausgebildet. Die erste Elektrode ist am distalen Ende des Katheters angeordnet ist und die Köperoberflächenelektrode an einer Körperoberfläche eines Patienten. Die erste Elektrode kann in einem Katheter angeordnet sein und aus dem Katheter am distalen Ende ragen. Der Katheter kann einen Schaft aufweisen, an dessen Ende die erste Elektrode angeordnet/angebracht ist.

Gemäß einem dritten Aspekt wird ein System zur irreversiblen Elektroporation eines Gewebes vorgeschlagen. Das System weist eine Vorrichtung gemäß dem ersten Aspekt zur gewebetypselektiven irreversiblen Elektroporation eines Gewebes und einen bipolaren Katheter auf. Der Katheter weist ein distales Ende auf. Das Elektrodenpaar ist als ein derartiges Elektrodenpaar ausgebildet ist, das an dem distalen Ende des bipolaren Katheters angeordnet ist. Das Elektrodenpaar kann in dem bipolaren Katheter angeordnet sein und aus dem Katheter am distalen Ende ragen.

Die Vorrichtung zur gewebetypselektiven irreversiblen Elektroporation eines Gewebes bietet den Vorteil, mit jeweils einem von sich unterscheidenden Katheter-Systemen kombiniert werden zu können. Die Vorrichtung kann sowohl unipolare als auch bipolare Multi-Elektroden Katheter-Systeme mit der gleichen Energie-Ansteuerung einsetzen, da sich Variationen in der Elektrodengröße sowie im Elektrodenabstand zwischen positivem und negativem Pol in der gemessenen Gewebe-Impedanz widerspiegeln und damit unabhängig von der Wahl des Katheters die gleiche maximal-Energie während der Ablation gewährleistet werden kann.

Gemäß einem fünften Aspekt wird ein Verfahren zur irreversiblen Elektroporation eines Gewebes vorgeschlagen. Das Verfahren umfasst ein Bereitstellen eines elektrischen Signalgenerators. Der Signalgenerator ist dazu eingerichtet, ein elektrisches Signal gemäß einem zu empfangenden Signal-Protokoll zu generieren und zu senden. Das Verfahren umfasst ein Bereitstellen eines Elektrodenpaars, das mit dem elektrischen Signalgenerator verbunden ist. Das Elektrodenpaar ist dazu eingerichtet, das elektrische Signal zu empfangen und eine elektrische Verbindung über das zwischen dem Elektrodenpaar liegende Gewebe zu schließen. Das Verfahren umfasst ein Bereitstellen einer mit dem Signalgenerator verbundenen Auswerte- und Steuereinheit. Das Verfahren umfasst ein Senden, in einer ersten Betriebsphase, eines Mess-Signal-Protokolls an den Signalgenerator mittels der Auswerte- und Steuereinheit. Das Verfahren umfasst ein Senden, in der ersten Betriebsphase, zumindest eines Mess-Signals mittels des Signalgenerators und des Elektrodenpaars durch das Gewebe. Das Verfahren umfasst ein Bestimmen, in der ersten Betriebsphase, einer Gewebeimpedanz auf Basis des durch das Gewebe gesendeten (und durch das Elektrodenpaar/die Auswerte- und Steuereinheit empfangenen) Mess-Signals. Das Verfahren umfasst ein Anpassen, in einer zweiten Betriebsphase, mittels der Auswerte- und Steuereinheit, zumindest eines Formats eines Burst-Signalfolge-Protokolls auf Basis der Gewebeimpedanz, um einen Energiebetrag pro Burst vorzugeben. Das Verfahren umfasst ein Senden, in einer dritten Betriebsphase, mittels der Auswerte und Steuereinheit, des angepassten Burst-Folge-Protokolls an den Signalgenerator.

Weitere Merkmale, Eigenschaften, Vorteile und mögliche Abwandlungen werden für einen Fachmann anhand der nachstehenden Beschreibungen deutlich, in der auf die beigefügten Zeichnungen Bezug genommen ist.
Fig.1 zeigt eine schematische Darstellung eines bipolaren Pulses.
Fig.2 zeigt eine schematische Darstellung eines beispielhaft umgesetzten Puls Protokolls, und
Fig.3 zeigt schematisch Verfahrensschritte zur irreversiblen Elektroporation eines Gewebes.

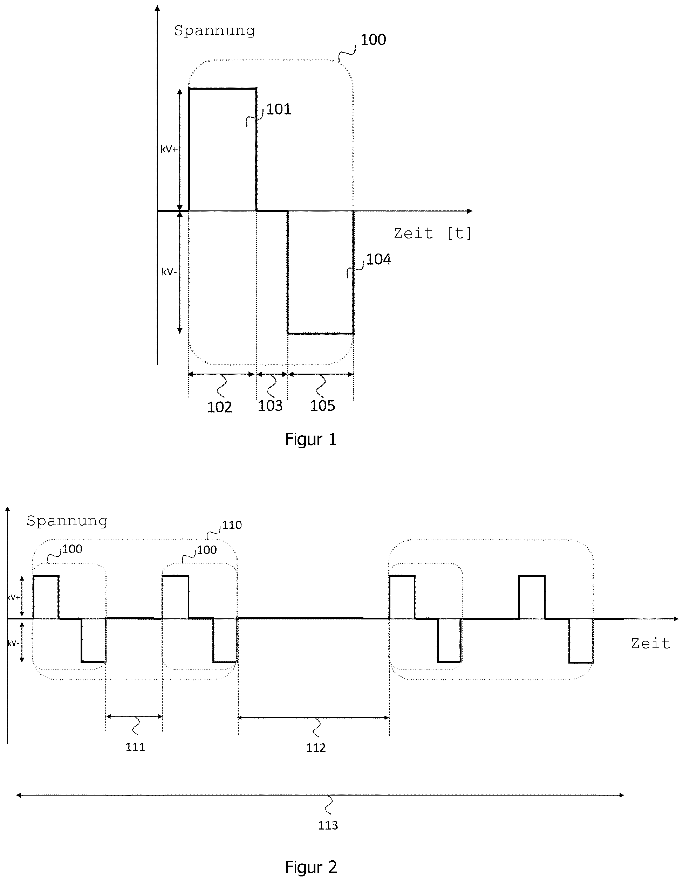

Figur 1 zeigt eine schematische Darstellung eines bipolaren Pulses 100, der von dem Signalgenerator generiert wird, wenn die Auswerte- und Steuereinheit ein Burst-Signalfolge-Protokoll an den Signalgenerator sendet. Der Signalgenerator ist im vorliegenden Fall als Spannungsquelle ausgebildet und nicht in der Figur 1 dargestellt. Im vorliegenden Beispiel sind die Formate, die Eigenschaften des bipolaren Pulses 100 bestimmen, durch einen Nutzer vordefiniert worden. Die Werte der Pulsauslenkung kV+, kV- des positiven 101 und negativen Pulses 104 sind im gezeigten Beispiel ±500 kV. Der dritte zeitliche Abstand 103 zwischen dem positiven 101 und negativen Puls 104 ist 2,5 µs. Die Pulsbreite 102 des positiven Pulses 101 unterscheidet sich von der Pulsbreite 105 des negativen Pulses 104. Der Unterschied der Pulsbreiten ist in der Figur 1 nicht dargestellt.

Soll in einer ersten Betriebsphase die Messung der Gewebeimpedanz erfolgen, wird ein Protokoll gemäß der oben erläuterten Formate von der Auswerte- und Steuereinheit an den Signalgenerator gesendet, der daraufhin das gesendete Mess-Signal-Protokoll umsetzt und ein Mess-Signal durch das Gewebe sendet. Im vorliegenden Beispiel wird als Mess-Signal der dargestellte bipolare Impuls 100 generiert und gesendet. Die Auswerte- und Steuereinheit bestimmt auf Basis des durch das Gewebe gesendeten bipolaren Pulses 100 eine Gewebeimpedanz. Die Gewebeimpedanz dient der Auswerte- und Steuereinheit als Basis für eine Anpassung der obigen Formate.

Im vorliegenden Fall ist das Gewebe ein myokardiales Gewebe und aus der Gewebeimpedanz wird abgeleitet, welche elektrische Feldstärke in das myokardiale Gewebe mindestens induziert werden muss, damit sich eine irreversible Elektroporation im myokardialen Gewebe einstellt.

Im vorliegenden Fall wird von der Auswerte- und Steuereinheit der dritte zeitliche Abstand 103 zwischen dem positiven 101 und negativen Puls 104 reduziert. Alternativ könnten die Pulsbreiten 102, 105 jeweils um einen unterschiedlichen Betrag vergrößert werden. Anders ausgedrückt sind die Pulsbreiten 102, 105, der dritte zeitlicher Abstand 103 und die Werte der Pulsauslenkung kV+, kV- jeweils unabhängig voneinander konfigurierbar.

Wurden die Formate durch die Auswerte- und Steuereinheit angepasst, wird in einer dritten Phase des Betriebs durch die Auswerte- und Steuereinheit eine Burst-Signalfolge-Protokoll an den als Spannungsquelle ausgebildeten Signalgenerator gesendet, der daraufhin eine Burst-Signalfolge zur irreversiblen Elektroporation durch das Gewebe sendet.

Figur 2 zeigt schematisch diese Burst-Signalfolge. Zu erkennen sind zwei Bursts, von denen einer mit dem Referenzzeichen 110 versehen ist. Jeder Burst weist zwei bipolare Pulse 100 auf. Jeder in der Burst-Signalfolge vorkommende bipolare Puls 100 weist die Eigenschaften auf, die das angepasste Format aus der vorhergehenden Figurenbeschreibung auf Basis der gemessen Gewebeimpedanz neu definiert/angepasst hat. Die erste Anzahl an Bursts, hier exemplarisch zwei Bursts, der zweite zeitlicher Abstand 111, und der erste zeitliche Abstand 112 zwischen zwei aufeinanderfolgenden Bursts 110, sind von einem Nutzer vor einer ersten Betriebsphase festgelegt worden. Die zu erkennende Burst-Signalfolge erstreckt sich über eine Dauer 113, die der Dauer der irreversiblen Elektroporation entspricht.

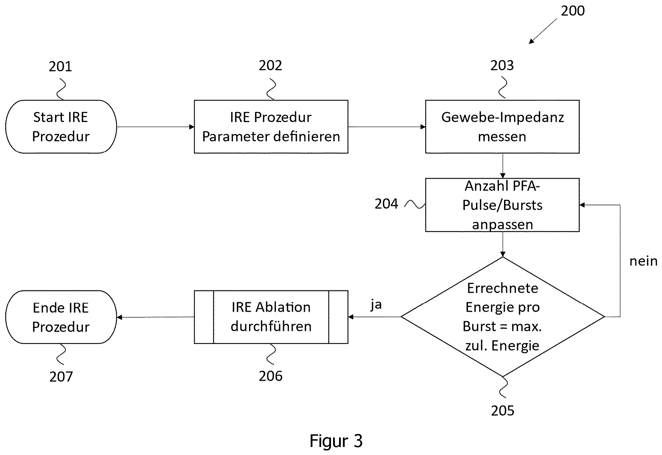

Figur 3 zeigt schematisch die Schritte, die das Verfahren 200 zur irreversiblen Elektroporation umfasst. Das Verfahren wird im Folgenden als irreversible Elektroporation-Prozedur oder kurz IRE-Prozedur bezeichnet. Die IRE-Prozedur startet mit dem Schritt 201. Folgend vordefiniert ein Nutzer, bspw. ein Arzt, im Schritt 202 die Werte der einzelnen Formate. Anders ausgedrückt, weist der Arzt jedem Format einen Wert zu. Dies kann durch manuelle Eingabe der entsprechenden Daten an der Auswerte- und Steuereinheit erfolgen. Die Werte der Formate und damit die Eigenschaft einer zu generierenden Signalfolge sind nun vordefiniert. In einem dritten Schritt erfolgt die Messung der Gewebeimpedanz. Dazu sendet die Auswerte- und Steuereinheit ein Mess-Signal-Protokoll an den Signalgenerator. Das Mess-Signal-Protokoll zeigt dem Signal-Generator an, mit welchen Werten die Formate umgesetzt werden sollen, die zu Generierung eines bipolaren Impulses nötig sind. Daraufhin wird von dem Signalgenerator ein Mess-Impuls durch das Gewebe gesendet und wieder empfangen. Auf Basis dieses empfangenen Mess-Impulses wird eine Gewebe-Impedanz gemessen, aus der abgeleitet werden kann, welche elektrische Feldstärke in das Gewebe induziert werden muss, damit sich eine IRE einstellt.

Für eine auf die Gewebe-Impedanzmessung folgende iterative Anpassung der Formate eines Burst-Signalfolge-Protokolls werden im Schritt 204 eine erste Anzahl an Bursts und eine zweite Anzahl von bipolaren Pulsen in den zuvor vom Nutzer definierten Formaten angepasst. Daraufhin wird eine Berechnung zur Ermittlung der Energie pro Burst durchgeführt 205, welche die maximal zulässige Energie für die noch folgenden Ablation nicht überschreitet aber die Ablation selbst sicherstellt. Mit anderen Worten folgt eine Anpassung zumindest eines oder einer Kombination von Formaten, sodass bspw. eine Verkohlung oder Wasserdampfbildung im Gewebe oder Nachbargewebe bei späterer Ablation vermieden wird, aber sich die IRE einstellt. Ist diese iterative Anpassung abgeschlossen, sendet die Auswerte- und Steuereinheit das angepasste Burst-Signal-Folgeprotokoll dem Signalgenerator, der daraufhin, nach Freigabe durch einen Nutzer bspw. Arzt, die Ablation 206 durchführt. Mit dem Abschluss der Ablation ist die IRE-Prozedur abgeschlossen 207.

## Patentansprüche

1. Eine Vorrichtung zur gewebetypselektiven irreversiblen Elektroporation eines Gewebes, aufweisend:
- einen elektrischen Signalgenerator, dazu eingerichtet, ein elektrisches Signal gemäß einem zu empfangenden Signal-Protokoll zu generieren und zu senden;
- ein Elektrodenpaar, das mit dem elektrischen Signalgenerator verbunden ist, dazu eingerichtet, das elektrische Signal zu empfangen und eine elektrische Verbindung über zwischen dem Elektrodenpaar liegendes Gewebe zu schließen;
- eine mit dem Signalgenerator verbundene Auswerte- und Steuereinheit, dazu eingerichtet:
- in einer ersten Betriebsphase dem Signalgenerator ein Mess-Signal-Protokoll zu senden und auf Basis von zumindest einem durch das Gewebe gesendeten Mess-Signal eine Gewebeimpedanz zu bestimmen,
- in einer zweiten Betriebsphase auf Basis der bestimmten Gewebeimpedanz zumindest ein Format eines Burst-Signalfolge-Protokolls anzupassen, um einen Energiebetrag pro Burst vorzugeben,
- in einer dritten Betriebsphase dem Signalgenerator das angepasste Burst-Signalfolge-Protokoll zu senden.

2. Die Vorrichtung nach Anspruch 1, wobei der vorgegebene Energiebetrag pro Burst, insbesondere in der dritten Betriebsphase, eine elektrische Feldstärke in das Gewebe induziert, die größer ist als eine für eine, insbesondere irreversible, Gewebeporung notwendige elektrische Feldstärke.

3. Die Vorrichtung nach Anspruch 1 oder 2, wobei die Formate des Burst-Signalfolge-Protokolls Eigenschaften und den Energiebetrag pro Burst einer durch den Signalgenerator zu generierende Burst-Signalfolge vorgeben, aufweisend:
- eine erste Anzahl an Bursts innerhalb der Burst-Signalfolge,
- zumindest einen ersten zeitlichen Abstand zwischen zumindest zwei aufeinanderfolgenden Bursts der Burst-Signalfolge,
- eine zweite Anzahl von bipolaren Pulsen innerhalb eines Bursts,
- zumindest einen zweiten zeitlichen Abstand zwischen zumindest zwei aufeinanderfolgenden bipolaren Pulsen innerhalb eines Bursts,
- einen dritten zeitlichen Abstand zwischen einem positiven und negativen Puls zumindest eines bipolaren Pulses,
- eine Pulsbreite eines positiven und/oder eines negativen Pulses zumindest eines bipolaren Pulses,
- einen Wert einer Pulsauslenkung eines positiven und/oder eines negativen Pulses zumindest eines bipolaren Pulses.

4. Die Vorrichtung nach einem der Ansprüche 1 bis 3, wobei das gesendete Mess-Signal einen Mess-Signalpegel aufweist, der einem Burst-Signalfolgepegel gleicht.

5. Ein System zur irreversiblen Elektroporation eines Gewebes, aufweisend die Vorrichtung nach einem der Ansprüche 1 bis 4 und einen monopolaren Katheter, wobei der Katheter ein distales Ende aufweist und das Elektrodenpaar als eine erste Elektrode und eine Köperoberflächenelektrode ausgebildet ist, wobei die erste Elektrode an dem distalen Ende des Katheters angeordnet ist und die Köperoberflächenelektrode an einer Körperoberfläche eines Patienten angeordnet ist.

6. Ein System zur irreversiblen Elektroporation eines Gewebes, aufweisend die Vorrichtung nach einem der Ansprüche 1 bis 4 und einen bipolaren Katheter, wobei der Katheter ein distales Ende aufweist und das Elektrodenpaar an dem distalen Ende angeordnet ist.

7. Ein Verfahren zur irreversiblen Elektroporation eines Gewebes, umfassend die Schritte:
- Bereitstellen eines elektrischen Signalgenerators, dazu eingerichtet, ein elektrisches Signal gemäß einem zu empfangenden Signal-Protokoll zu generieren und zu senden;
- Bereitstellen eines Elektrodenpaars, das mit dem elektrischen Signalgenerator verbunden ist, dazu eingerichtet, das elektrische Signal zu empfangen und eine elektrische Verbindung über das zwischen dem Elektrodenpaar liegende Gewebe zu schließen;
- Bereitstellen einer mit dem Signalgenerator verbundenen Auswerte- und Steuereinheit;
- in einer ersten Betriebsphase, Senden eines Mess-Signal-Protokolls an den Signalgenerator mittels der Auswerte- und Steuereinheit;
- in der ersten Betriebsphase, Senden zumindest eines Mess-Signals mittels des Signalgenerators und des Elektrodenpaars durch das Gewebe;
- in der ersten Betriebsphase, Bestimmen einer Gewebeimpedanz auf Basis des durch das Gewebe gesendeten Mess-Signals;
- in einer zweiten Betriebsphase, mittels der Auswerte- und Steuereinheit, Anpassen zumindest eines Formats eines Burst-Signalfolge-Protokolls auf Basis der Gewebeimpedanz, um einen Energiebetrag pro Burst vorzugeben;
- in einer dritten Betriebsphase, mittels der Auswerte und Steuereinheit, Senden des angepassten Burst-Folge-Protokolls an den Signalgenerator.

8. Das Verfahren nach Anspruch 7, wobei die Formate des Burst-Signalfolge-Protokolls Eigenschaften und den Energiebetrag pro Burst einer durch den Signalgenerator zu generierende Burst-Signalfolge vorgeben, aufweisend:
- eine erste Anzahl an Bursts innerhalb der Burst-Signalfolge,
- zumindest einen ersten zeitlichen Abstand zwischen zumindest zwei aufeinanderfolgenden Bursts der Burst-Signalfolge,
- eine zweite Anzahl von bipolaren Pulsen innerhalb eines Bursts,
- zumindest einen zweiten zeitlichen Abstand zwischen zumindest zwei aufeinanderfolgenden bipolaren Pulsen innerhalb eines Bursts,
- einen dritten zeitlichen Abstand zwischen einem positiven und negativen Puls zumindest eines bipolaren Pulses,
- eine Pulsbreite eines positiven und/oder eines negativen Pulses zumindest eines bipolaren Pulses, und
- einen Wert einer Pulsauslenkung eines positiven und/oder eines negativen Pulses zumindest eines bipolaren Pulses.
